(19) Europäisches Patentamt
European Patent Office
Office européen des brevets

(11) Veröffentlichungsnummer: **0 450 487 A1**

# (12) EUROPÄISCHE PATENTANMELDUNG

(21) Anmeldenummer: **91104870.0**

(22) Anmeldetag: **27.03.91**

(51) Int. Cl.⁵: **C07D 413/04**, C07D 413/12, A61K 31/535

(30) Priorität: **31.03.90 DE 4010488**

(43) Veröffentlichungstag der Anmeldung: **09.10.91 Patentblatt 91/41**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Englert, Heinrich Christian, Dr.**
**Stormstrasse 13**
**W-6238 Hofheim am Taunus(DE)**
Erfinder: **Mania, Dieter, Dr.**
**Goethestrasse 43**
**W-6240 Königstein am Taunus(DE)**
Erfinder: **Linz, Wolfgang, Dr.**
**Huxelrebenweg 54**
**W-6500 Mainz(DE)**

(54) **Benzoxazinderivate, Verfahren zu ihrer Herstellung und ihre Verwendung zum Behandeln oder zur Prophylaxe von Krankheiten.**

(57) Benzoxazin-Derivate der Formel I

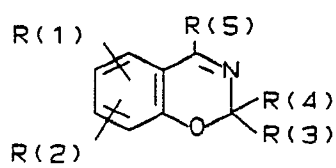

I

mit R(1) und R(2) gleich H, OH, Alkoxy, Hal oder einer Vielzahl anderer Substituenten,
R(3) gleich Alkyl, Alkanoyl;
R(4) gleich H, Alkyl, Alkanoyl, und
R(5) Pyrid(azin)yloxy, Pyrimidinyloxy, Pyridazinyloxy, sowie weiteren Heterocyclen
und ihre Salze sind wertvolle Arzneimittel zur Behandlung von Störungen des cardiovaskulären Systems, von Asthma, von glattmuskulären Störungen, des Bluthochdrucks, von Herzrhythmusstörungen, von Durchblutungsstörungen des Gehirns; sie dienen ferner zur topischen Anwendung zur Bekämpfung des Haarausfalls.

EP 0 450 487 A1

Die Erfindung betrifft Verbindungen I

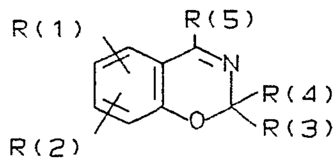

I

in welchen bedeuten:

R(1) und R(2), die gleich sind oder verschieden:

a) Wasserstoff, ($C_1$-$C_4$)-Alkyl, OH, ($C_1$-$C_4$)-Alkoxy, Cl, Br oder

b) einer der beiden Substituenten R(1) und R(2) hat die unter a) angegebene Bedeutung und der andere CHO, ($C_1$-$C_4$)-Alkylcarbonyl, ($C_1$-$C_4$)-Alkyl-CS, ($C_1$-$C_4$)-Alkyl-OOC, ($C_1$-$C_4$)-Alkoxy-CS, ($C_1$-$C_4$)-AlkylenCOO, ($C_1$-$C_4$)-Alkylen-CS-O, Hydroxy-($C_1$-$C_4$)-Alkyl, HS-($C_1$-$C_4$)-Alkyl-, $NO_2$, $NH_2$, ($C_1$-$C_4$)-Alkylen-NH, Di-($C_1$-$C_4$)-Alkylamino, CN, Cl, Br, J, $CF_3$, ($C_1$-$C_4$)-AlkylSO, ($C_1$-$C_4$)-Alkyl$SO_2$, ($C_1$-$C_4$)-Alkoxy-SO, ($C_1$-$C_4$)-Alkoxy-$SO_2$, ($C_1$-$C_4$)-Alkanoyl-NH, Benzamido, ($C_1$-$C_4$)-Alkoxy-CO-NH, $H_2NSO$, ($C_1$-$C_4$)-Alkyl-NHSO, $H_2NSO_2$, ($C_1$-$C_4$)AlkylNH$SO_2$, Di($C_1$-$C_4$)-AlkylN$SO_2$, $H_2NCO$, ($C_1$-$C_4$)-AlkylNHCO, Di-($C_1$-$C_4$)-AlkylNCO, $H_2NCS$, ($C_1$-$C_4$)-AlkylNHCS, Di-($C_1$-$C_4$)-Alkyl-NCS, ($C_1$-$C_4$)-AlkylSONH, ($C_1$-$C_4$)-Alkyl-$SO_2$NH, ($C_1$-$C_4$)-AlkoxySONH, ($C_1$-$C_4$)-Alkyloxy-$SO_2$NH, Nitro-($C_1$-$C_4$)-Alkyl, Cyan-($C_1$-$C_4$)-Alkyl bedeutet

oder

c) R(1) und R(2)

Ar-, ArO, ArCO, ArCS, ArSO, Ar$SO_2$, ArOSO, ArO$SO_2$, ArNHSO, ArNH$SO_2$, ArNHCO, ArNHCS, ArSONH Ar$SO_2$NH, ArOSONH, Ar$SO_2$NH sind,

worin Ar ein aromatisches oder heteroaromatisches System ist, welches unsubstituiert ist oder substituiert ist mit 1 bis 3 gleichen oder verschiedenen Resten ($C_1$-$C_2$)-Alkyl, ($C_1$-$C_2$)-Alkoxy, F, Cl, Br, J, $CF_3$, CN, $NO_2$, CO($C_1$-$C_2$)Alkyl, $SO_p$($C_1$-$C_2$)-Alkyl mit p für eins oder Zwei;

R(3) ($C_1$-$C_4$)-Alkyl, ($C_1$-$C_4$)-Alkanoyl;

R(4) Wasserstoff, ($C_1$-$C_4$)-Alkyl, ($C_1$-$C_4$)-Alkanoyl;

R(3) und R(4) gemeinsam auch ($C_3$-$C_6$)-Alkylen;

R(5) unsubstituiertes oder ein- oder zweifach durch ($C_1$-$C_4$)-Alkyl, ($C_1$-$C_4$)-Alkylen, ($C_1$-$C_4$)-Alkanoyl, F, Cl, Br, J, OH, O($C_1$-$C_4$)-Alkyl, O($C_1$-$C_4$)-Alkylen, O($C_1$-$C_4$)-Alkanoyl, $NO_2$, $NH_2$, ($C_1$-$C_4$)-AlkanoylNH, HOOC oder ($C_1$-$C_4$)-AlkylOOC, substituiertes Pyridyl-oxy, Pyridazinyl-oxy, Pyrimidinyl-oxy, Pyrazinyl-oxy, Oxo-dihydro-pyridyl-oxy, Oxo-dihydro-pyridazinyl-oxy, Oxo-dihydro-pyrimidinyl-oxy, Oxo-dihydro-pyrazinyl-oxy, 1H-2-Pyridon-1-yl, 1H-6-Pyridazinon-1-yl, 1H-2-Pyrimidinon-1-yl, 1H-6-Pyrimidinon-1-yl, 1H-2-Pyrazinon-1-yl, 1H-2-Thiopyridon-1yl ist, wobei die Reste auch vollständig oder partiell hydriert sein können,

R(5) ferner für einen Ring

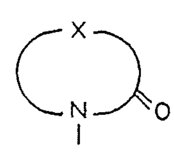

steht, in welchem X eine Kette $(CH_2)_m$ bedeutet mit m gleich zwei bis vier, welche unsubstituiert ist oder substituiert durch 1 - 3 ($C_1$-$C_2$)-Alkylgruppen, und welche $(CH_2)_m$-Kette durch ein Heteroatom Y in der Bedeutung S, O, NR(6) unterbrochen sein kann, wobei R(6) für Wasserstoff oder ($C_1$-$C_4$)-Alkyl steht,

sowie deren pharmazeutisch akzeptable Salze.

Bevorzugt sind Verbindungen I mit

R(1) und R(2) gleich Wasserstoff, Methyl, Ethyl, Methoxy, Ethoxy, Acetyl, Propionyl, Thioacetyl, Thiopropionyl, Methoxycarbonyl, Ethoxycarbonyl, Methoxythiocarbonyl, Ethoxythiocarbonyl, Acetoxy, Propionoxy, Thioacetoxy, Thiopropionoxy, Hydroxymethyl, 1-Hydroxyethyl, 2-Hydroxyethyl, Mercaptomethyl, 1-Mercaptoethyl, 2-Mercaptoethyl, Methylamino, Ethylamino, Dimethylamino, Diethylamino, Methylsulfinyl, Ethylsulfinyl, Methylsulfonyl, Ethylsulfonyl, Methoxysulfinyl, Ethoxysulfinyl, Methoxysulfonyl, Ethoxysulfonyl, Acetamido, Formamido, Propionamido, Benzamido, Methoxycarbonylamino, Ethoxycarbonylamino, Methylaminosulfinyl, Ethylaminosulfinyl, Methylaminosulfonyl, Ethylaminosulfonyl, N-Methylcarbamoyl, N-Ethylcarbamoyl, N,N-Dimethylcarbamoyl, N,N-Diethylcarbamoyl, N-Methylthiocarbamoyl, N-Ethylthiocarbamoyl, N,N-Dimethylthiocarbamoyl, N,N-Diethylthiocarbamoyl, Methylsulfinylamino, Ethylsulfinylamino, Methylsulfonylamino, Ethylsulfonylamino, Methoxysulfonylamino, Ethoxysulfonylamino, Nitromethyl, 2-Nitroethyl, Cyanmethyl, 1-Cyanethyl, 2-Cyanethyl; Phenyl, 4- oder 2-Chlorphenyl, o- oder p-Tolyl, Phenoxy, 4- oder 2-Chlorphenoxy, o- oder p-Tolyloxy, Benzoyl, 4- oder 2-Chlorbenzoyl, o- oder p-Toluoyl, Benzylsulfinyl, 4- oder 2-Chlorbenzsulfinyl, o- oder p-Tolylsulfinyl, Benzsulfonyl, 4- oder 2-Chlorbenzsulfonyl, o- oder p-Tolylsulfonyl, Benzoxysulfinyl, 4- oder 2-Chlorbenzoxysulfinyl, o- oder p-Tolyloxysulfinyl, Benzaminosulfinyl, 4- oder 2-Chlorbenzaminosulfinyl, o- oder p-Tolylaminosulfinyl, Benzaminosulfonyl, 4- oder 2-Chlorbenzaminosulfonyl, o- oder p-Tolylaminosulfonyl, N-Phenylcarbamoyl, N-[4- oder 2-]Chlorphenylcarbamoyl, o- oder p-Tolylcarbamoyl, N-Phenyl-thiocarbamoyl, N-[4- oder 2-]Chlorphenyl-thiocarbamoyl, o- oder p-Tolyl-thiocarbamoyl, Phenylsulfinylamino, 4- oder 2-Chlorphenylsulfinylamino, o- oder p-Tolylsulfinylamino, Phenylsulfonylamino, 4- oder 2-Chlorphenylsulfonylamino, o- oder p-Tolylsulfonylamino, Phenoxysulfinylamino, 4- oder 2-Chlorphenoxysulfinylamino, o- oder p-Tolyloxysulfinylamino, Phenoxysulfonylamino, 4- oder 2-Chlorphenoxysulfonylamino, o- oder p-Tolyloxysulfonylamino;

R(3) $(C_1-C_4)$-Alkyl;
R(4) Wasserstoff oder $(C_1-C_4)$-Alkyl;
R(5) wie oben definiert.

Ganz besonders bevorzugt sind Verbindungen I, in denen bedeuten:

R(1) Wasserstoff, Cl, Br, $NO_2$, CN, $(C_1-C_4)$-Alkoxycarbonyl, $(C_1-C_4)$-Alkylthio, Phenylsulfonyl;
R(2) gleich Wasserstoff;
R(3) und R(4) $(C_1-C_4)$-Alkyl;
R(5) 1H-2-Pyridon-1-yl, 2-Oxo-pyrrolidin-1-yl, 2-Oxopiperidin-1-yl, die unsubstituiert sind oder durch $(C_1-C_4)$-Alkyl substituiert.

Insbesondere bevorzugt sind Verbindungen I mit:

R(1) gleich $NO_2$, CN, Phenylsulfonyl,
R(2) gleich Wasserstoff
R(3) und R(4) gleich $(C_1-C_2)$-Alkyl
R(5 ) 2-Oxopyrrolidinyl.

Die Einführung eines oder mehrerer Substituenten in den 2-Oxopyrrolidinylring erzeugt ein Asymmetrie- bzw. mehrere Asymmetriezentren. Die erfindungsgemäßen Verbindungen können dann nicht nur als Antipoden auftreten, sondern bei mehreren Substituenten auch als Diastereomere.

Der Erfindung lag die Aufgabe zugrunde, neue Verbindungen mit wertvollen Eigenschaften aufzufinden, die als Arzneimittel verwendet werden können.

Überraschenderweise wurde eine Gruppe neuer 1,3-Benzoxazin-Derivate der Formel I mit wertvollen pharmakologischen Eigenschaften gefunden. Diese Verbindungen zeigen eine sehr gute relaxierende Wirkung an der glatten Muskulatur. Sie können daher sowohl als Bronchodilatoren bei der Behandlung von Atmungsstörungen, insbesondere bei Bronchialasthma als auch zur Behandlung des Bluthochdrucks eingesetzt werden. Sie eignen sich ferner zur Behandlung von Kontraktionsstörungen der glatten Muskulatur des Gastrointestinaltrakts, des Uterus, des Harnleiters, einschließlich dem Ureter. Die Störungen schließen vorzeitige Wehen, Inkontinenz der Harnblase, Nierenkolik, sowie die Passage von Nierensteinen ein. Die Verbindungen können ferner bei kardiovaskulären Störungen angewendet werden, die nicht mit dem Blutdruck zusammenhängen, wie zur Behandlung und/oder Prophylaxe des pulmonaren Bluthochdrucks; außerdem bei Durchblutungsstörungen des Gehirns, und zur Behandlung von Haarausfall (topische Anwendung), sie können beispielsweise eine antiarrhythmische oder cardioprotektive Wirkung zeigen.

Die Erfindung betrifft auch ein Verfahren zur Herstellung der Verbindung I, nach welchem man Verbindungen der Formel II

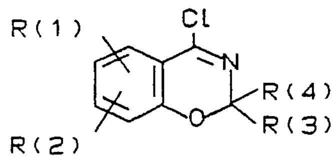

II ,

in der die Reste R(1) bis R(4) die oben genannte Bedeutung haben und deren Herstellung teilweise im J. Med. Chem. 1987, 30, 205-208 und in Heterocycles Vol. 16, 1165 (1981) beschrieben worden sind,

a) mit R(5)H (III), worin R(5) die in der Formel I angegebene Bedeutung hat, oder mit einem ihrer reaktionsfähigen Derivate umsetzt.

Vorzugsweise werden die Verbindungen der Formel I durch Reaktion von Verbindungen der Formel II mit Verbindungen der Formel III in einem inerten Lösungsmittel bei 0 - 150 ° C hergestellt.

Es ist zweckmäßig, in Gegenwart einer Base zu arbeiten. Als Base eignen sich Alkali- oder Erdalkalihydroxide, -hydride oder auch -amide, wie NaOH, KOH, $Ca(OH)_2$, NaH, $NaNH_2$, ferner organische Basen wie Triethylamin oder Pyridin.

Als inerte Lösungsmittel eignen sich Ether wie Diethylether, Dioxan, Tetrahydrofuran, Amide wie Dimethylformamid, Sulfoxide wie Dimethylsulfoxid. Die Reaktionsprodukte können ohne Schwierigkeit getrennt und isoliert werden, z.B. durch Kristallisation und/oder Chromatographie.

Weiterhin kann man den Rest R(1) der Formel I in einen anderen Rest umwandeln.
Beispielsweise ist es möglich, daß man ein H-Atom [R(1) = H] mittels Nitrierung durch eine Nitrogruppe ersetzt und/oder eine Nitrogruppe zu einer Aminogruppe reduziert oder eine Cyangruppe einführt, indem ein Halogenderivat der Formel I beispielsweise R(1) oder R(2) = Br mit CuCN umsetzt.

Eine Nitrierung gelingt unter den üblichen Bedingungen z.B. mit einem Gemisch aus konzentrierter $HNO_3$ und konzentrierter $H_2SO_4$ in Eisessig bei Temperaturen von 0 bis 30 ° C.

Die Einführung einer Cyangruppe gelingt durch Umsetzung der entsprechenden 6-Br-Benzoxazinverbindungen mit Cu-I-cyanid in Dimethylformamid bei Temperaturen von 120 - 160 ° C;

b) oder die Verbindungen der Formel II mit Verbindungen der Formel IV

$$H_2N-(CH_2)_{n+2}\underset{\underset{O}{\|}}{C}-L \qquad (IV)$$

zu Verbindungen der Formel V

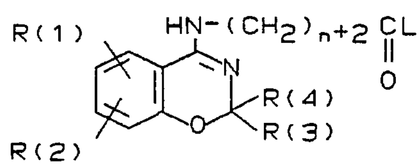

umsetzt, in denen R(1) - R(4) wie oben definiert sind und L die Bedeutung einer Fluchtgruppe hat, und diese cyclisiert zu Verbindungen der Formel VI

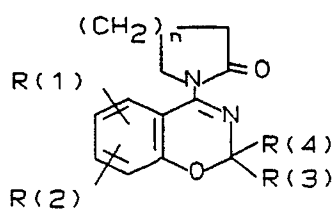

VI

mit n = 1-2.

Die erfindungsgemäßen Verbindungen der Formel I sind, wie bereits erwähnt, Antihypertensiva. Sie werden ferner bei der Therapie und/oder Prophylaxe von Störungen des cardiovaskulären Systems, insbesondere bei dekompensierter Herzinsuffizienz, Angina pectoris sowie peripheren oder cerebralen Gefäßerkrankungen angewendet. Ferner können sie bei Erkrankungen, die mit Veränderungen der nicht-vaskulären Muskulatur zusammenhängen, wie Erkrankungen der Atmungswege, Asthma, Inkontinenz der Harnblase, angewendet werden.

Die Verbindungen der Formel I können in der Human- und Veterinärmedizin eingesetzt werden. Sie werden dabei in Dosierungen von mindestens 0.001 mg/kg/Tag, vorzugsweise 0.005 mg/kg/Tag und insbesondere 0.05 mg/kg/Tag bis maximal 10 mg/kg/Tag, vorzugsweise 5 mg/kg/Tag und insbesondere 2 mg/kg/Tag als Kapseln, Dragees, Tabletten, Puder, Zäpfchen, Lösungen, Spray oder Aerosol mit Zusätzen oder ohne Zusätze von galenischen Hilfsstoffen enteral, z.B. oral oder parenteral z.B. intravenös verabfolgt, jeweils bezogen auf ein Gewicht von 75 kg. Die neuen Verbindungen können auch lyophilisiert und die erhaltenen Lyophilisate z.B. zur Herstellung von Injektionspräparaten verwendet werden.

**BEISPIELE**

**Herstellung des Ausgangsmaterials:**

a) In einer Lösung aus 65 ml Dimethylformamid und 160 ml 2,2-Dimethoxypropan werden 219 g (1,6 Mol) Salicylsäureamid eingerührt und mit 0,2 g p-Toluolsulfonsäure 6 Stunden unter Stickstoff auf Rückfluß erhitzt. Nach üblicher Aufarbeitung erhält man 3,4-Dihydro-2,2-dimethyl-4-oxo-2H-1,3-benzoxazin.

Schmp.: 137° C (aus Methyl-tert.-butylether).

b) Eine Lösung von 53,1 g (0,3 Mol) des vorstehenden 1,3-Benzoxazins in einer Mischung aus 62,3 g (0,3 Mol) Phosphorpentachlorid und 28 ml Phosphoroxychlorid wird eine Stunde bei 20° C gerührt. Nachdem die heftige HCl-Entwicklung vorüber war, wurde noch 2 Stunden auf 50° C erhitzt und anschließend das Phosphoroxychlorid im Wasserstrahlvakuum abdestilliert. Nach Destillation im Hochvakuum erhält man 4-Chlor-2,2-dimethyl-2H-1,3-benzoxazin vom Schmp. 85 - 90° C /0,05 mmHg.

Analog erhält man:

6-Brom-4-chlor-2,2-dimethyl-2H-1,3-benzoxazin,
4,6-Dichlor-2,2-dimethyl-2H-1,3-benzoxazin,
4-Chlor-2,2-diethyl-2H-1,3-benzoxazin,
4,6-Dichlor-2,2-diethyl-2H-1,3-benzoxazin.

**Beispiel 1**

2,2-Diethyl-4-(2'-oxo-pyrrolidin-1'-yl)-2H-1,3-benzoxazin

Zu einer Suspension von 2,4 g (0,08 Mol) Natriumhydrid (80 %ig) in 50 ml DMSO wurde unter Rühren langsam eine Lösung von 6,8 g (0,08 Mol) Pyrrolidin-2-on in 25 ml DMSO eingetragen. Anschließend tropften bei 20° C 8,9 g (0,04 Mol) 4-Chlor-2,2-diethyl-1,3-benzoxazin zu. Nach Stehen über Nacht wurde das Reaktionsprodukt noch 2 Stunden auf 50° C erhitzt und nach Abkühlen in Eiswasser eingetragen. Der Niederschlag wurde abgesaugt, getrocknet und aus Petrolether umkristallisiert.

Kristalle vom Schmp. 58° C.

**Beispiel 2**

2,2-Diethyl-4-(3'-methyl-2'-oxo-pyrrolidin-1'-yl)-2H-1,3-benzoxazin

Analog Beispiel 1 mit 7,9 g (0,08 Mol) 3-Methylpyrrolidin-2-on. Das Endprodukt wurde in heißem Petrolether gelöst und mit Aktivkohle gereinigt. Beim Erkalten fiel die Titelverbindung kristallin aus.

Kristalle vom Schmp. 69 - 70° C.

**Beispiel 3**

2,2-Diethyl-4-(4'-methyl-2'-oxo-pyrrolidin-1'-yl)-2H-1,3-benzoxazin

Analog Beispiel 1 mit 7,9 g (0,08 Mol) 4-Methylpyrrolidin-2-on. Das Reaktionsprodukt wurde nach Eintragen in Eiswasser mit Methylenchlorid extrahiert; die organische Phase wurde getrocknet, eingedampft und der Rückstand über eine Kieselgelsäule mit Dichlormethan/Ethylacetat/Ethanol 94:3:3 chromatographiert.
Kristalle vom Schmp. 75 - 76° C (aus Petrolether)

**Beispiel 4**

6-Chlor-2,2-diethyl-4-(2'-oxo-pyrrolidin-1'-yl)-2H-1,3-benzoxazin

Zu einer Suspension von 0,6 g (0,02 Mol) Natriumhydrid (80 %ig) in 10 ml DMSO wurden unter Stickstoff 1,6 ml (0,02 Mol) Pyrrolidin-2-on zugetropft. Nach 30minütigem Rühren bei 20° C tropften 5,2 g (0,02 Mol) 4,6-Dichlor-2,2-diethyl-2H-1,3-benzoxazin zu. Der Ansatz blieb über Nacht stehen und wurde anschließend in Eiswasser eingetragen und mehrfach mit Methylenchlorid extrahiert. Die vereinigten organischen Phasen wurden getrocknet, eingedampft, und der Rückstand wurde über eine Kieselgelsäule mit Petrolether/Ethylacetat 3:1 chromatographiert. Die Titelverbindung wurde aus wenig Petrolether umkristallisiert.
Kristalle vom Schmp. 53° C

**Beispiel 5**

6-Chlor-2,2-diethyl-4-(3'-methyl-2'-oxo-pyrrolidin-1'-yl)-2H-1,3-benzoxazin

Analog Beispiel 4 mit 2 g (0.02 Mol) 3-Methylpyrrolidin-2-on. Die Ausbeuten wurden verbessert, indem man die Natriumhydridsuspension in DMSO mit dem 3-Methylpyrrolidin-2-on über Nacht stehen ließ und erst danach unter Stickstoff 6,4 g (0.02 Mol) 4,6-Dichlor-2,2-diethyl-2H-1,3-benzoxazin zutropfen ließ. Es wurde 6 Stunden nachgerührt und in Eiswasser eingetragen. Beim Anreiben mit einem Glasstab kristallisierte 6-Chlor-2,2-diethyl-4-(3'-methyl-2'-oxo-pyrrolidin-1'-yl)-2H-1,3-benzoxazin. Es wurde abgesaugt, getrocknet, in heißem Petrolether gelöst und mit Aktivkohle geklärt. Beim Einengen fiel die Titelverbindung kristallin aus.
Kristalle vom Schmp. 67-68° C.

**Beispiel 6**

2,2-Diethyl-6-nitro-4-(3'-methyl-2'-oxo-pyrrolidin-1'-yl) 2H-1,3-benzoxazin

Zu einer Lösung von 5,73 g (0,02 Mol) 2,2-Diethyl-4-(3'-methyl-2'-oxo-pyrrolidin-1'-yl)-2H-1,3-benzoxazin in 60 ml Eisessig wurden 5 ml (~ 0,1 Mol) Salpetersäure (mehr als 90 %ig) zugetropft. Nach Zugabe von 20 ml konz. $H_2SO_4$ wurde der Ansatz 5 Minuten auf 60° C erhitzt, anschließend wurde auf 20° C abgekühlt, in Eiswasser eingetragen und mit Dichlormethan extrahiert. Die vereinigten organischen Phasen wurden mit Wasser neutral gewaschen, eingeengt und der Rückstand über eine Kieselgelsäule mit Petrolether/Ethylacetat 3:1 chromatographiert.
Kristalle vom Schmp. 91-92° C (aus Petrolether).

**Beispiel 7**

6-Amino-2,2-diethyl-4-(3'-methyl-2'-oxo-pyrrolidin-1'-yl) 2H-1,3-benzoxazin

Zu einer Lösung von 3,3 g (0,01 Mol) 2,2-Diethyl-4-(3'-methyl-2'-oxopyrrolidin-1'-yl)-6-nitro-2H-1,3-benzoxazin wurde eine Suspension von 0,3 g Pd/C (10 %) in 20 ml Wasser zugegeben und unter Normalbedingungen hydriert. Nach der berechneten Wasserstoffaufnahme wurde mit Stickstoff gespült, der Katalysator abgesaugt, das Filtrat eingeengt und der Rückstand aus Diisopropylether umkristallisiert.
Kristalle vom Schmp. 69-72° C.

**Beispiel 8**

2,2-Dimethyl-4-(2'-oxo-pyrrolidin-1'-yl)-2H-1,3-benzoxazin

Zu einer Suspension von 4,5 g (0,15 Mol) Natriumhydrid (80 %ig) in 45 ml DMSO wurden vorsichtig 11,4 ml (0.15 Mol) Pyrrolidin-2-on zugetropft. Der Ansatz wurde über Nacht stehengelassen, anschließend tropften unter Rühren 19,6 g (0,1 Mol) 4-Chlor-2,2-dimethyl-2H-1,3-benzoxazin zu. Nach sechsstündigem Rühren wurde in Eiswasser eingetragen, der Niederschlag mit Wasser neutral gewaschen, in Dichlormethan gelöst, mit Aktivkohle geklärt, eingeengt und aus Diisopropylether umkristallisiert.
Kristalle vom Schmp. 125-126 °C.

**Beispiel 9**

2,2-Dimethyl-4-(3'-methyl-2'-oxo-pyrrolidin-1'-yl)-2H-1,3-benzoxazin

Analog Beispiel 8 mit 14,9 g (0,15 Mol) 3-Methyl-pyrrolidin-2-on
Kristalle vom Schmp. 105 °C.

**Beispiel 10**

6-Chlor-2,2-dimethyl-4-(2'-oxo-pyrrolidin-1'-yl)-2H-1,3-benzoxazin

Analog Beispiel 8 mit 23 g (0,1 Mol) 4,6-Dichlor-2,2-dimethyl-2H-1,3-benzoxazin
Kristalle vom Schmp. 119 °C (aus Petrolether)

**Beispiel 11**

6-Brom-2,2-dimethyl-4-(2'-oxo-pyrrolidin-1'-yl)-2H-1,3-benzoxazin

Analog Beispiel 8 mit 27,4 g (0,1 Mol) 6-Brom-4-chlor-2,2-dimethyl-2H-1,3-benzoxazin
Kristalle vom Schmp. 120 °C (aus Diisopropylether)

**Beispiel 12**

2,2-Dimethyl-6-nitro-4-(2'-oxo-pyrrolidin-1'-yl)-2H-1,3-benzoxazin

Zu einer Lösung von 3,2 g (0,013 Mol) 2,2-Diethyl-4-(2'-oxo-pyrrolidin-1'-yl)-2H-1,3-benzoxazin in 32 ml konz. Schwefelsäure und 32 ml Eisessig wurden bei 20 °C 5 ml rauchende Salpetersäure zugetropft. Nach zweistündigem Rühren wurde der Ansatz in Eiswasser eingetragen und mehrfach mit je 30 ml Dichlormethan extrahiert. Die vereinigten organischen Phasen wurden mit Wasser annähernd neutral gewaschen, getrocknet, eingeengt und der Rückstand über eine Kieselgelsäule mit Petrolether/Ethylacetat 3:1 gereinigt.
Kristalle vom Schmp. 142-143 °C (aus Diethylether)

**Beispiel 13**

6-Brom-2,2-dimethyl-4-(3'-methyl-2'-oxo-pyrrolidin-1'-yl)-2H-1,3-benzoxazin

Zu einer Suspension von 3 g (0,1 Mol) Natriumhydrid (80%ig) in 60 ml DMSO wurden unter Stickstoff 9,9 g (0,1 Mol) 3-Methylpyrrolidin-2-on eingetragen und mehrere Stunden bei 20 °C stehen gelassen. Anschließend wurden unter Rühren 22 g (0,08 Mol) 6-Brom-4-chlor-2,2-dimethyl-2H-1,3-benzoxazin in kleinen Portionen zugefügt und weitere vier Stunden nachgerührt. Das Reaktionsprodukt wurde dann in Eiswasser eingetragen und mehrfach mit 50 ml Dichlormethan extrahiert. Die vereingigten Extrakte wurden mit Wasser gewaschen, getrocknet, eingedampft und der Rückstand über eine Kieselgelsäule mit Petrolether/Ethylacetat 3:1 von Verunreinigungen getrennt.
Kristalle vom Schmp. 110-111 °C (aus wenig Diisopropylether)

**Beispiel 14**

6-Cyano-2,2-dimethyl-4-(2'-oxo-pyrrolidin-1'-yl)-2H-1,3-benzoxazin

Zu einer Lösung von 2,6 g (0,08 Mol) 6-Brom-2,2-dimethyl-4-(2'-oxopyrrolidin-1'-yl)-2H-1,3-benzoxazin in 25 ml DMF wurden 0,9 g (0,1 Mol) Kupfer(I)-cyanid eingetragen und 16 Stunden unter Stickstoff auf

150 °C erhitzt. Nach dem Abkühlen auf 20 °C wurde das Reaktionsprodukt in eiskalte konz. Ammoniaklösung eingerührt und mit Dichlormethan und Diethylether extrahiert. Die vereinigten organischen Phasen wurden mit Wasser gewaschen, getrocknet, eingeengt und der Rückstand über eine Kieselgelsäule mit Petrolether/Ethylacetat 1:1 chromatographiert. Die Titelverbindung kristallisierte aus Diisopropylether.
Kristalle vom Schmp. 182-183 °C.

**Beispiel 15**

6-Cyano-2,2-dimethyl-4-(3'-methyl-2'-oxo-pyrrolidin-1'-yl)-2H-1,3-benzoxazin

Analog Beispiel 14 mit 6,7 g (0,02 Mol) 6-Brom-2,2-dimethyl-4-(3'-methyl-2'-oxopyrrolidin-1'-yl)-2H-1,3-benzoxazin und 1,8 g (0,02 Mol) Kupfer(I)-cyanid in 65 ml DMF. Nach Eintragen in die kalte konz. Ammoniaklösung wurde mehrfach mit Dichlormethan extrahiert. Die vereinigten organischen Phasen wurden mit Wasser neutral gewaschen, getrocknet, filtriert, eingeengt und der Rückstand aus heißem Diisopropylether umkristallisiert.
Kristalle vom Schmp. 116 °C.

**Patentansprüche**

1. Verbindungen I

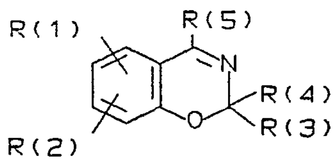

I

in welchen bedeuten:
R(1) und R(2), die gleich sind oder verschieden:
a) Wasserstoff, $(C_1$-$C_4)$-Alkyl, OH, $(C_1$-$C_4)$-Alkoxy, Cl, Br oder
b) einer der beiden Substituenten R(1) und R(2) hat die unter a) angegebene Bedeutung
und der andere CHO, $(C_1$-$C_4)$-Alkylcarbonyl, $(C_1$-$C_4)$-Alkyl-CS, $(C_1$-$C_4)$-Alkyl-OOC, $(C_1$-$C_4)$-Alkoxy-CS, $(C_1$-$C_4)$-AlkylenCOO, $(C_1$-$C_4)$-Alkylen-CS-O, Hydroxy-$(C_1$-$C_4)$-Alkyl, HS-$(C_1$-$C_4)$-Alkyl-, $NO_2$, $NH_2$, $(C_1$-$C_4)$-Alkylen-NH, Di-$(C_1$-$C_4)$-Alkylamino, CN, Cl, Br, J, $CF_3$, $(C_1$-$C_4)$-AlkylSO, $(C_1$-$C_4)$-AlkylSO$_2$, $(C_1$-$C_4)$-Alkoxy-SO, $(C_1$-$C_4)$-Alkoxy-SO$_2$, $(C_1$-$C_4)$-Alkanoyl-NH, Benzamido, $(C_1$-$C_4)$-Alkoxy-CO-NH, $H_2NSO$, $(C_1$-$C_4)$-Alkyl-NHSO, $H_2NSO_2$, $(C_1$-$C_4)$AlkylNHSO$_2$, Di$(C_1$-$C_4)$-AlkylNSO$_2$, $H_2NCO$, $(C_1$-$C_4)$-AlkylNHCO, Di-$(C_1$-$C_4)$-AlkylNCO, $H_2NCS$, $(C_1$-$C_4)$-AlkylNHCS, Di-$(C_1$-$C_4)$-Alkyl-NCS, $(C_1$-$C_4)$-AlkylSONH, $(C_1$-$C_4)$-AlkylSO$_2$NH, $(C_1$-$C_4)$-AlkoxySONH, $(C_1$-$C_4)$-Alkyloxy-SO$_2$NH, Nitro-$(C_1$-$C_4)$-Alkyl, Cyan-$(C_1$-$C_4)$-Alkyl
bedeutet
oder
c) R(1) und R(2)
Ar-, ArO, ArCO, ArCS, ArSO, $ArSO_2$, ArOSO, $ArOSO_2$, ArNHSO, $ArNHSO_2$, ArNHCO, ArNHCS, ArSONH, $ArSO_2NH$, ArOSONH, $ArSO_2NH$ sind,
worin Ar ein aromatisches oder heteroaromatisches System ist, welches unsubstituiert ist oder substituiert ist mit 1 bis 3 gleichen oder verschiedenen Resten $(C_1$-$C_2)$-Alkyl, $(C_1$-$C_2)$Alkoxy, F, Cl, Br, J, $CF_3$, CN, $NO_2$, CO$(C_1$-$C_2)$Alkyl, $SO_p(C_1$-$C_2)$-Alkyl mit p für eins oder zwei;

| | |
|---|---|
| R(3) | $(C_1$-$C_4)$Alkyl, $(C_1$-$C_4)$-Alkanoyl; |
| R(4) | Wasserstoff, $(C_1$-$C_4)$Alkyl, $(C_1$-$C_4)$-Alkanoyl; |
| R(3) und R(4) | gemeinsam auch $(C_3$-$C_6)$-Alkylen; |
| R(5) | unsubstituiertes oder ein- oder zweifach durch $(C_1$-$C_4)$Alkyl, $(C_1$-$C_4)$-Alkylen, $(C_1$-$C_4)$-Alkanoyl, F, Cl, Br, J, OH, O$(C_1$-$C_4)$-Alkyl, O$(C_1$-$C_4)$-Alkylen, O$(C_1$-$C_4)$-Alkanoyl, $NO_2$, $NH_2$, $(C_1$-$C_4)$-AlkanoylNH, HOOC oder $(C_1$-$C_4)$-AlkylOOC, |

substituiertes Pyridyl-oxy, Pyridazinyl-oxy, Pyrimidinyl-oxy, Pyrazinyl-oxy, Oxo-dihydro-pyridyl-oxy, Oxo-dihydro-pyridazinyl-oxy, Oxo-dihydro-pyrimidinyl-oxy,

Oxo-dihydro-pyrazinyl-oxy, 1H-2-Pyridon-1yl, 1H-6-Pyridazinon-1-yl, 1H-2-Pyrimidinon-1-yl, 1H-6-Pyrimidinon-1-yl, 1H-2-Pyrazinon-1-yl, 1H-2-Thiopyridon-1yl ist, wobei die Reste auch vollständig oder partiell hydriert sein können,

R(5) ferner für einen Ring

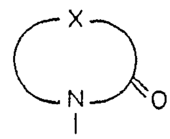

steht, in welchem X eine Kette $(CH_2)_m$ bedeutet mit m gleich zwei bis vier, welche unsubstituiert ist oder substituiert durch 1 - 3 $(C_1\text{-}C_2)$-Alkylgruppen, und welche $(CH_2)_m$-Kette durch ein Heteroatom Y in der Bedeutung S, O, NR(6) unterbrochen sein kann, wobei R(6) für Wasserstoff oder $(C_1\text{-}C_4)$-Alkyl steht,

sowie deren pharmazeutisch akzeptable Salze.

**2.** Verbindungen I nach Anspruch 1, dadurch gekennzeichnet, daß R(1) und R(2) gleich Wasserstoff, Methyl, Ethyl, Methoxy, Ethoxy, Acetyl, Propionyl, Thioacetyl, Thiopropionyl, Methoxycarbonyl, Ethoxycarbonyl, Methoxythiocarbonyl, Ethoxythiocarbonyl, Acetoxy, Propionoxy, Thioacetoxy, Thiopropionoxy, Hydroxymethyl, 1-Hydroxyethyl, 2-Hydroxyethyl, Mercaptomethyl, 1-Mercaptoethyl, 2-Mercaptoethyl, Methylamino, Ethylamino, Dimethylamino, Diethylamino, Methylsulfinyl, Ethylsulfinyl, Methylsulfonyl, Ethylsulfonyl, Methoxysulfinyl, Ethoxysulfinyl, Methoxysulfonyl, Ethoxysulfonyl, Acetamido, Formamido, Propionamido, Benzamido, Methoxycarbonylamino, Ethoxycarbonylamino, Methylaminosulfinyl, Ethylaminosulfinyl, Methylaminosulfonyl, Ethylaminosulfonyl, N-Methylcarbamoyl, N-Ethylcarbamoyl, N,N-Dimethylcarbamoyl, N,N-Diethylcarbamoyl, N-Methylthiocarbamoyl, N-Ethylthiocarbamoyl, N,N-Dimethylthiocarbamoyl, N,N-Diethylthiocarbamoyl, Methylsulfinylamino, Ethylsulfinylamino, Methylsulfonylamino, Ethylsulfonylamino, Methoxysulfonylamino, Ethoxysulfonylamino, Nitromethyl, 2-Nitroethyl, Cyanmethyl, 1-Cyanethyl, 2-Cyanethyl; Phenyl, 4- oder 2-Chlorphenyl, o- oder p-Tolyl, Phenoxy, 4- oder 2-Chlorphenoxy, o- oder p-Tolyloxy, Benzoyl, 4- oder 2-Chlorbenzoyl, o- oder p-Toluoyl, Benzylsulfinyl, 4- oder 2-Chlorbenzsulfinyl, o- oder p-Tolylsulfinyl, Benzsulfonyl, 4- oder 2-Chlorbenzsulfonyl, o- oder p-Tolylsulfonyl, Benzoxysulfinyl, 4- oder 2-Chlorbenzoxysulfinyl, o- oder p-Tolyloxysulfinyl, Benzaminosulfinyl, 4- oder 2-Chlorbenzaminosulfinyl, o- oder p-Tolylaminosulfinyl, Benzaminosulfonyl, 4- oder 2-Chlorbenzaminosulfonyl, o- oder p-Tolylaminosulfonyl, N-Phenylcarbamoyl, N-[4- oder 2-]Chlorphenylcarbamoyl, o- oder p-Tolylcarbamoyl, N-Phenyl-thiocarbamoyl, N-[4- oder 2-]Chlorphenylthiocarbamoyl, o- oder p-Tolyl-thiocarbamoyl, Phenylsulfinylamino, 4- oder 2-Chlorphenylsulfinylamino, o- oder p-Tolylsulfinylamino, Phenylsulfonylamino, 4- oder 2-Chlorphenylsulfonylamino, o- oder p-Tolylsulfonylamino, Phenoxysulfinylamino, 4- oder 2-Chlorphenoxysulfinylamino, o- oder p-Tolyloxysulfinylamino, Phenoxysulfonylamino, 4- oder 2-Chlorphenoxysulfonylamino, o- oder p-Tolyloxysulfonylamino;

R(3) $(C_1\text{-}C_4)$-Alkyl;
R(4) Wasserstoff oder $(C_1\text{-}C_4)$-Alkyl;
R(5) wie in Anspruch 1 definiert.

**3.** Verbindungen I nach Anspruch 1, dadurch gekennzeichnet, daß

R(1) Wasserstoff, Cl, Br, $NO_2$, CN, $(C_1\text{-}C_4)$-Alkoxycarbonyl, $(C_1\text{-}C_4)$-Alkylthio, Phenylsulfonyl;
R(2) gleich Wasserstoff;
R(3) und R(4) $(C_1\text{-}C_4)$-Alkyl;
R(5) 1H-2-Pyridon-1-yl, 2-Oxo-pyrrolidin-1-yl, 2-Oxopiperidin-1-yl, die unsubstituiert sind oder durch $(C_1\text{-}C_4)$-Alkyl substituiert,

sind.

**4.** Verbindungen I nach Anspruch 1, dadurch gekennzeichnet, daß

R(1) gleich $NO_2$, CN, Phenylsulfonyl,
R(2) gleich Wasserstoff
R(3) und R(4) gleich $(C_1\text{-}C_2)$-Alkyl
R(5) 2-Oxopyrrolidinyl

sind.

**5.** Verfahren zum Herstellen einer Verbindung I nach Anspruch 1, dadurch gekennzeichnet, daß man Verbindungen der Formel II

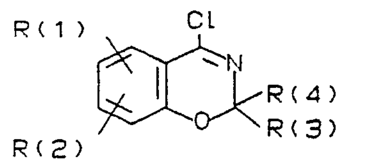

II ,

in der die Reste R(1) bis R(4) die oben genannte Bedeutung haben,

a) mit R(5)H (III), worin R(5) die in der Formel I angegebene Bedeutung hat, oder mit einem ihrer reaktionsfähigen Derivate umsetzt,

oder daß man

b) oder die Verbindungen der Formel II mit Verbindungen der Formel IV

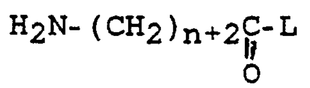

(IV)

zu Verbindungen der Formel V

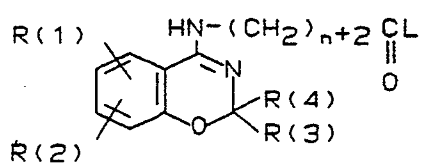

umsetzt, in denen R(1) - R(4) wie in Anspruch 1 definiert sind und L die Bedeutung einer Fluchtgruppe hat,

und diese cyclisiert zu Verbindungen der Formel VI

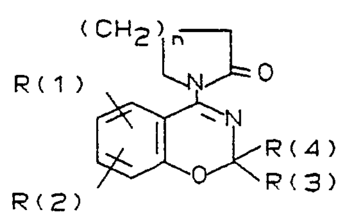

VI

mit n = 1 - 2.

**6.** Verbindung I nach Anspruch 1 zur Verwendung als Medikament zur Therapie und Prophylaxe von Störungen des cardiovaskulären Systems.

**7.** Verfahren zum Behandeln von Störungen des cardiovaskulären Systems durch Applizieren einer geeigneten Menge einer Verbindung I nach Anspruch 1.

**8.** Verwendung einer Verbindung nach Anspruch 1 als Heilmittel zur Therapie von Herz-Kreislauferkrankungen.

**9.** Verwendung einer Verbindung nach Anspruch 1 als Heilmittel gegen Asthma.

**10.** Verwendung einer Verbindung nach Anspruch 1 als Heilmittel gegen glattmuskuläre Störungen.

**11.** Verwendung einer Verbindung nach Anspruch 1 als Heilmittel zur Bekämpfung des Bluthochdrucks.

**12.** Verwendung einer Verbindung nach Anspruch 1 als Heilmittel zur Cardioprotektion und gegen Herzrhythmusstörungen.

**13.** Verwendung einer Verbindung nach Anspruch 1 als Heilmittel gegen Durchblutungsstörungen des Gehirns.

**14.** Topische Anwendung einer Verbindung I nach Anspruch 1 zur Bekämpfung des Haarausfalls.

**Patentansprüche für folgende Vertragsstaaten: ES, GR**

**1.** Verfahren zum Herstellen einer Verbindung I

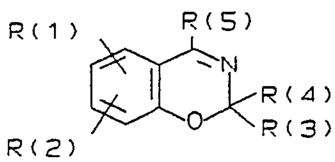

I

in welcher bedeuten:
R(1) und R(2), die gleich sind oder verschieden:
a) Wasserstoff, $(C_1-C_4)$-Alkyl, OH, $(C_1-C_4)$-Alkoxy, Cl, Br oder
b) einer der beiden Substituenten R(1) und R(2) hat die unter a) angegebene Bedeutung und der andere CHO, $(C_1-C_4)$-Alkylcarbonyl, $(C_1-C_4)$-Alkyl-CS, $(C_1-C_4)$-Alkyl-OOC, $(C_1-C_4)$-Alkoxy-CS, $(C_1-C_4)$-AlkylenCOO, $(C_1-C_4)$-Alkylen-CS-O, Hydroxy-$(C_1-C_4)$-Alkyl, HS-$(C_1-C_4)$-Alkyl, $NO_2$, $NH_2$, $(C_1-C_4)$-Alkylen-NH, Di-$(C_1-C_4)$-Alkylamino, CN, Cl, Br, J, $CF_3$, $(C_1-C_4)$-AlkylSO, $(C_1-C_4)$-Alkyl$SO_2$, $(C_1-C_4)$-Alkoxy-SO, $(C_1-C_4)$-Alkoxy-$SO_2$, $(C_1-C_4)$-Alkanoyl-NH, Benzamido, $(C_1-C_4)$-Alkoxy-CO-NH, $H_2NSO$, $(C_1-C_4)$-Alkyl NHSO, $H_2NSO_2$, $(C_1-C_4)$Alkyl$NHSO_2$, Di$(C_1-C_4)$-Alkyl$NSO_2$, $H_2NCO$, $(C_1-C_4)$-AlkylNHCO, Di-$(C_1-C_4)$-AlkylNCO, $H_2NCS$, $(C_1-C_4)$-AlkylNHCS, Di-$(C_1-C_4)$-Alkyl-NCS, $(C_1-C_4)$-Alkyl-SONH, $(C_1-C_4)$-Alkyl$SO_2NH$, $(C_1-C_4)$-AlkoxySONH, $(C_1-C_4)$-Alkyl-$SO_2NH$, Nitro-$(C_1-C_4)$-Alkyl, Cyan-$(C_1-C_4)$-Alkyl
bedeutet
oder
c) R(1) und R(2)
Ar-, ArO, ArCO, ArCS, ArSO, $ArSO_2$, ArOSO, $ArOSO_2$, ArNHSO, $ArNHSO_2$, ArNHCO, ArNHCS, ArSONH, $ArSO_2NH$, ArOSONH, $ArSO_2NH$ sind,
worin Ar ein aromatisches oder heteroaromatisches System ist, welches unsubstituiert ist oder substituiert ist mit 1 bis 3 gleichen oder verschiedenen Resten $(C_1-C_2)$-Alkyl, $(C_1-C_2)$-Alkoxy, F, Cl, Br, J, $CF_3$, CN, $NO_2$, $CO(C_1-C_2)$Alkyl, $SO_p(C_1-C_2)$-Alkyl mit p für eins oder zwei;

| | |
|---|---|
| R(3) | $(C_1-C_4)$-Alkyl, $(C_1-C_4)$-Alkanoyl; |
| R(4) | Wasserstoff, $(C_1-C_4)$-Alkyl, $(C_1-C_4)$-Alkanoyl; |
| R(3) und R(4) | gemeinsam auch $(C_3-C_6)$-Alkylen; |
| R(5) | unsubstituiertes oder ein- oder zweifach durch $(C_1-C_4)$-Alkyl, $(C_1-C_4)$-Alkylen, $(C_1-C_4)$-Alkanoyl, F, Cl, Br, J, OH, $O(C_1-C_4)$-Alkyl, $O(C_1-C_4)$-Alkylen, $O(C_1-C_4)$-Alkanoyl, $NO_2$, $NH_2$, $(C_1-C_4)$-AlkanoylNH, HOOC oder $(C_1-C_4)$-AlkylOOC,<br><br>substituiertes Pyridyl-oxy, Pyridazinyl-oxy, Pyrimidinyl-oxy, Pyrazinyl-oxy, Oxo-dihydro-pyridyl-oxy, Oxo-dihydro-pyridazinyl-oxy, Oxo-dihydro-pyrimidinyl-oxy, Oxo-dihydro-pyrazinyl-oxy, 1H-2-Pyridon-2yl, 1H-6-Pyridazinon-1-yl, 1H-2-Pyrimidinon-1-yl, 1H-6-Pyrimidinon-1-yl, 1H-2-Pyrazinon-1-yl, 1H-2-Thiopyridon-1yl ist, wobei die Reste auch vollständig oder partiell hydriert sein können, |
| R(5) | ferner für einen Ring |

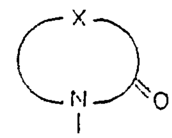

steht, in welchem X eine Kette $(CH_2)_m$ bedeutet mit m gleich zwei bis vier, welche unsubstituiert ist oder substituiert durch 1 - 3 $(C_1-C_2)$-Alkylgruppen, und welche $(CH_2)_m$-Kette durch ein Heteroatom Y in der Bedeutung S, O, NR(6) unterbrochen sein kann, wobei R(6) für Wasserstoff oder $(C_1-C_4)$-Alkyl steht,

sowie von deren pharmazeutisch akzeptablen Salzen, dadurch gekennzeichnet, daß man Verbindungen der Formel II

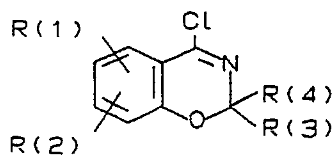

II ,

in der die Reste R(1) bis R(4) die oben genannte Bedeutung haben,

a) mit R(5)H (III), worin R(5) die in der Formel I angegebene Bedeutung hat, oder mit einem ihrer reaktionsfähigen Derivate umsetzt,

oder daß man

b) oder die Verbindungen der Formel II mit Verbindungen der Formel IV

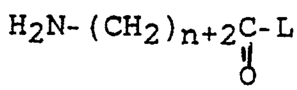

(IV)

zu Verbindungen der Formel V

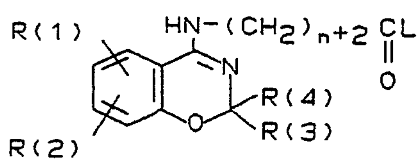

umsetzt, in denen R(1) - R(4) wie oben definiert sind und L die Bedeutung einer Fluchtgruppe hat,

und diese cyclisiert zu Verbindungen der Formel VI

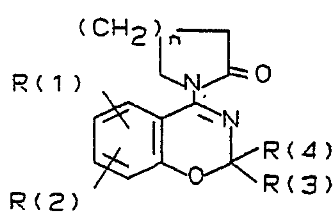

VI

mit n = 1 - 2.

2. Verfahren nach Anspruch 1, dadurch gekennzeichnet, daß die Substituenten folgende Bedeutung haben:
R(1) und R(2) Wasserstoff, Methyl, Ethyl, Methoxy, Ethoxy, Acetyl, Propionyl, Thioacetyl, Thiopropionyl, Methoxycarbonyl, Ethoxycarbonyl, Methoxythiocarbonyl, Ethoxythiocarbonyl, Acetoxy, Propionoxy, Thioacetoxy, Thiopropionoxy, Hydroxymethyl, 1-Hydroxyethyl, 2-Hydroxyethyl, Mercaptomethyl, 1-Mercaptoethyl, 2-Mercaptoethyl, Methylamino, Ethylamino, Dimethylamino, Diethylamino, Methylsulfinyl, Ethylsulfinyl, Methylsulfonyl, Ethylsulfonyl, Methoxysulfinyl, Ethoxysulfinyl, Methoxysulfonyl, Ethoxysulfonyl, Acetamido, Formamido, Propionamido, Benzamido, Methoxycarbonylamino, Ethoxycarbonylamino, Methylaminosulfinyl, Ethylaminosulfinyl, Methylaminosulfonyl, Ethylaminosulfonyl, N-Methylcarbamoyl, N-Ethylcarbamoyl, N,N-Dimethylcarbamoyl, N,N-Diethylcarbamoyl, N-Methylthiocarbamoyl, N-Ethylthiocarbamoyl, N,N-Dimethylthiocarbamoyl, N,N-Diethylthiocarbamoyl, Methylsulfinylamino, Ethylsulfinylamino, Methylsulfonylamino, Ethylsulfonylamino, Methoxysulfonylamino, Ethoxysulfonylamino, Nitromethyl, 2-Nitroethyl, Cyanmethyl, 1-Cyanethyl, 2-Cyanethyl; Phenyl, 4- oder 2-Chlorphenyl, o- oder p-Tolyl, Phenoxy, 4- oder 2-Chlorphenoxy, o- oder p-Tolyloxy, Benzoyl, 4- oder 2-Chlorbenzoyl, o- oder p-Toluoyl, Benzylsulfinyl, 4- oder 2-Chlorbenzsulfinyl, o- oder p-Tolylsulfinyl, Benzsulfonyl, 4- oder 2-Chlorbenzsulfonyl, o- oder p-Tolylsulfonyl, Benzoxysulfinyl, 4- oder 2-Chlorbenzoxysulfinyl, o- oder p-Tolyloxysulfinyl, Benzaminosulfinyl, 4- oder 2-Chlorbenzaminosulfinyl, o- oder p-Tolylaminosulfinyl, Benzaminosulfonyl, 4- oder 2-Chlorbenzaminosulfonyl, o- oder p-Tolylaminosulfonyl, N-Phenylcarbamoyl, N-[4- oder 2-]Chlorphenylcarbamoyl, o- oder p-Tolylcarbamoyl, N-Phenyl-thiocarbamoyl, N-[4- oder 2-]Chlorphenyl-thiocarbamoyl, o- oder p-Tolyl-thiocarbamoyl, Phenylsulfinylamino, 4- oder 2-Chlorphenylsulfinylamino, o- oder p-Tolylsulfinylamino, Phenylsulfonylamino, 4- oder 2-Chlorphenylsulfonylamino, o- oder p-Tolylsulfonylamino, Phenoxysulfinylamino, 4- oder 2-Chlorphenoxysulfinylamino, o- oder p-Tolyloxysulfinylamino, Phenoxysulfonylamino, 4- oder 2-Chlorphenoxysulfonylamino, o- oder p-Tolyloxysulfonylamino;

| | |
|---|---|
| R(3) | $(C_1\text{-}C_4)$-Alkyl; |
| R(4) | Wasserstoff oder $(C_1\text{-}C_4)$-Alkyl; |
| R(5) | wie in Anspruch 1 definiert. |

3. Verfahren nach Anspruch 1, dadurch gekennzeichnet, daß die Substituenten folgende Bedeutung haben:

| | |
|---|---|
| R(1) | Wasserstoff, Cl, Br, $NO_2$, CN, $(C_1\text{-}C_4)$-Alkoxycarbonyl, $(C_1\text{-}C_4)$-Alkylthio, Phenylsulfonyl; |
| R(2) | Wasserstoff; |
| R(3) und R(4) | $(C_1\text{-}C_4)$-Alkyl; |
| R(5) | 1H-2-Pyridon-1-yl, 2-Oxo-pyrrolidin-1-yl, 2-Oxopiperidin-1-yl, die unsubstituiert sind oder durch $(C_1\text{-}C_4)$-Alkyl substituiert. |

4. Verfahren nach Anspruch 1, dadurch gekennzeichnet, daß die Substituenten folgende Bedeutung haben:

| | |
|---|---|
| R(1) | $NO_2$, CN, Phenylsulfonyl, |
| R(2) | Wasserstoff |
| R(3) und R(4) | $(C_1\text{-}C_2)$-Alkyl |
| R(5) | 2-Oxopyrrolidinyl. |

5. Verfahren zum Behandeln von Störungen des cardiovaskulären Systems durch Applizieren einer geeigneten Menge einer Verbindung I nach Anspruch 1.

6. Verwendung einer Verbindung nach Anspruch 1 als Heilmittel zur Therapie von Herz-Kreislauferkrankungen.

7. Verwendung einer Verbindung nach Anspruch 1 als Heilmittel gegen Asthma.

8. Verwendung einer Verbindung nach Anspruch 1 als Heilmittel gegen glattmuskuläre Störungen.

9. Verwendung einer Verbindung nach Anspruch 1 als Heilmittel zur Bekämpfung des Bluthochdrucks.

10. Verwendung einer Verbindung nach Anspruch 1 als Heilmittel zur Cardioprotektion und gegen Herzrhythmusstörungen.

11. Verwendung einer Verbindung nach Anspruch 1 als Heilmittel gegen Durchblutungsstörungen des Gehirns.

12. Topische Anwendung einer Verbindung I nach Anspruch 1 zur Bekämpfung des Haarausfalls.

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

Nummer der Anmeldung
EP 91104870.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 98, Nr. 3, 17. Januar 1983, Columbus, Ohio, USA SANKYO CO., LTD. "4-Amino-1,3-benzoxazine derivatives" Seite 514, Spalte 1, Zusammenfassung-Nr. 16 704s & Jpn. Kokai Tokkyo Koho JP 57,130,979 -- | 1-6 | C 07 D 413/04<br>C 07 D 413/12<br>A 61 K 31/535 |
| X | CHEMICAL ABSTRACTS, Band 97, Nr. 1, 5. Juli 1982, Columbus, Ohio, USA SANKYO CO., LTD. "1,3-Benzoxazines" Seite 612, Spalte 2, Zusammenfassung-Nr. 6 314e & Jpn. Kokai Tokkyo Koho JP 82 07,482 -- | 1-6 | |
| X | CHEMICAL ABSTRACTS, Band 96, Nr. 19, 10. Mai 1982, Columbus, Ohio, USA | 1-5 | RECHERCHIERTE SACHGEBIETE (Int. Cl.4)<br>C 07 D 413/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: —
Nicht recherchierte Patentansprüche: 7-14
Grund für die Beschränkung der Recherche:

Art. 52(4) Verfahren zur therapeutischen Behandlung des menschlichen oder tierischen Körpers.

| Recherchenort | Abschlußdatum der Recherche | Prüfer |
|---|---|---|
| WIEN | 03-06-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82

Europäisches Patentamt

**EUROPÄISCHER TEILRECHERCHENBERICHT**

Nummer der Anmeldung
-2-
EP 91104870.0

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| | TACHIKAWA, RYUJI et al. "Studies on 1,3-benzoxazines. III. Reaction of imidoyl chlorides of 1,3-benzoxazines with 2-hydroxy- or 2-mercaptopyridine N-oxides: a novel sulfur-nitrogen bond formation via elctrocyclic rearrangement" Seite 677, Spalte 2, Zusammenfassung-Nr. 161 856f & Chem. Pharm. Bull. 1981, 29(12), 3529-35 -- | | |
| X | CHEMICAL ABSTRACTS, Band 95, Nr. 13, 28. September 1981, Columbus, Ohio, USA SANKYO CO., LTD. "1,3-Benzoxazine derivatives" Seite 711, Spalte 1, Zusammenfassung-Nr. 115 572s & Jpn. Kokai Tokkyo Koho 81 43,281 -- | 1-6 | RECHERCHIERTE SACHGEBIETE (Int. Cl. 4) |
| X | CHEMICAL ABSTRACTS, Band 93, Nr. 7, 18. August 1980, Columbus, Ohio, USA WACHI, KAZUYUKI et al. "Studies of 1,3-benzoxazines. I. Synthesis of primary 2-aminopyrides via the reaction of imidoyl chlorides of 1,3-benzoxazines with pyridine N-oxides" Seite 970, Spalte 1, Zusammenfassung-Nr. 71 667d & Chem. Pharm. Bull. 1980, 28(2), 465-72 ---- | 1-5 | |